# EUROPEAN PATENT APPLICATION

(11) **EP 2 219 127 A1**
(43) Date of publication of application: **18.08.2010**
(21) Application number: 10153242.2
(22) Date of filing: 10.02.2010
(51) Int. Cl.: G06F 19/00

(54) **System for generating a health profile from available input data concerning a patient, and transforming such health profile into relevant health information in human intelligible form**

(30) Priority: 11.02.2009 US 151812 P; 14.01.2010 CA 2690222
(71) Applicant: MediResource Inc., Toronto, ON M3C 1T5 (CA)
(72) Inventor: Kostoff, Paul, Toronto Ontario M2K 3A7 (CA)
(74) Representative: Schlich, George William

(57) **Abstract**

An automated system for use by pharmacies, clinics or health care institutions which provides health information that comprises disease state information, the system having: a clinic computer having access to medical information containing a medication profile for the patient, and a remote computer having access to a drug-disease database. The disease state information is determined by a process where the clinic computer analyzes the medication information, creating a data packet comprising drug products being used, associating a unique identification number (UTDN) to the patient and data packet, and transmitting the UTDN and data packet to the remote computer. The remote computer then cross-referencing the drug products contained within said data packet with individual disease states and identifying disease state information that is relevant to be provided to a patient.

## Description

### Background Of The Invention

The present invention relates to the healthcare field and in particular to the area of the electronic provision of health care information to a patient.

Health professionals receive a significant healing opportunity by involving the patient in their own treatment. It has often been observed that informed patients get healthier faster and stay healthier longer. Historically, a patient has been dependent on his/her healthcare worker for access to information. In a pharmacy setting, a pharmacist can provide a consultation to a patient receiving a prescription on the drug product being dispensed, possible side effects and answer any questions a patient might have. In an institutional setting such as a hospital, a health care provider such as a nurse or doctor can also provide consultation services to a patient seeking information. In many instances, the provision of this information is limited due to the time constraints that the health care provider has or limited professional knowledge.

US Patent No. 7,267,278 describes an electronic pharmacy system that distributes information on pharmaceutical products.

US Patent No. 6,206,829 describes a computerized medical diagnostic and treatment advice system in which a patient directly answers questions posed to him/her by a computer system that results in a medical diagnostic or treatment advice.

US Patent No. 6,375,469 describes a system in which entertainment content and personalized health information is presented to a patient in a television or web browser, the health information being derived from detailed information such as a medical treatment regimen, health profile, educational treatment plan, etc. that are maintained in a health care provider server.

US Patent No. 6,240,394 describes a system used in pharmacies where advisory messages which includes information pertaining to the dispensed product based on the identification of a drug product based on the drug product's label. This system can also be used to generate promotional material or information on health in general.

US Published Application No. 2006/0266,826 teaches a system for use in a pharmacy setting where a pharmacy publication system uses de-identified patient information.

None of the systems in the prior art provide for the unique system of the current invention that allows both institutional and pharmacy health care providers/workers to provide automated and relevant disease related information to a patient derived in a simple and straightforward manner as part of a customized printout.

### Summary of the Invention

This invention relates generally to the field of healthcare and specifically to an automated system to distribute health information e.g. through health facilities such as physician offices, clinics, hospitals, pharmacies, chiropractic offices and/or dental offices. The system generally provides in one aspect of the invention, a manner in which a patient's health is improved by providing relevant information about a condition or treatment to the patient.

In another aspect of the invention, the system need not be restricted to the pharmacy, such as the installed base of retail pharmacies, but can also be more broadly implemented to a wider variety of locals and in particular health care institutions such as health clinics, physician offices, hospitals, chiropractic offices and/or dental offices where patients are normally discharged or released with effective follow-up mechanisms.

The system also provides in another aspect of the invention, an electronic medical record systems which facilitates the wide spread usage by health care institutions and pharmacies.

In another aspect of the invention, the system can satisfy a patients' need to be empowered and informed in the management of their personal health. For example, the system can enable the health care system and health care professionals to take the lead in being the primary provider of health information and allow them to keep up with the demand by patients for health information. The resulting benefit is that patients become informed about their condition and are not tempted to try dubious or inappropriate treatments because of lack of knowledge, or lack of adequate supervision by their health care professional. For example, patients will no longer be forced to turn to the internet for information because their physician, nurse or pharmacist does not have enough time or knowledge to adequately inform or counsel patients.

In another aspect of the invention, a technology solution is provided which can offer an improved method to implement efficient delivery of health information to patients thereby allowing in one embodiment of the invention, clinics, to have effective and economical technologies in place to deliver health information to patients.

In yet another aspect of the invention, time can be saved by the user and embodiments achieve the result that front line health care providers have the time for adequately informing their patients, at the same time realizing the goal without requiring reasonable but generally unaffordable compensation for spending time counseling patients. In addition, it is possible that one can avoid the need to find space to store and organize a wide range of patient health information, and thus overcome a serious barrier to furnishing relevant and adequate information to patients with a wide range of medical conditions. Also other challenges such as educating staff and poor communication skills can be overcome.

In yet another aspect of the invention, significant economic benefits can be realized when it is feasible for health care professionals to adequately and efficiently educate patients about their health and the importance of complying with their treatment. Well informed patients are healthier and consume less health care resources. Well informed patients make fewer visits to hospital emergency services, consume fewer medications and are less of a burden on caregivers. Patients generally want their doctor, pharmacist or nurse to be their primary source of health information; they trust them more than all other sources of health information.

In still yet another aspect of the invention the system can effectively meet the challenge of implementing an efficient and economical computer system for health practitioners in spite of the current diversity of existing software and operating systems. This removes a significant barrier to the successful distribution of health information e.g. by providing a 'one size fits all' solution that is economically feasible to implement. Exemplary embodiments disclosed herein have overcome this barrier by developing a platform-independent, centralized or remote system that integrates seamlessly with existing clinic hardware and software. This allows distribution to a broader range of health clinics.

In another aspect, the present invention is directed to an automated system for use by pharmacies, clinics or health care institutions, said system for providing health information to a patient, said system comprising:
- a clinic computer having access to medical information for said patient, said medical information comprising at least a medication profile for said patient,
- a remote computer having access to a drug-disease database
   said clinic and remote computers being networkably connected so as to allow data transfer between said computers,
- said health information comprising disease state information, said disease state information being determined by
   ○ said clinic computer,
      ■ analyzing said medication information for said patient
      ■ creating a data packet comprising drug products being used by said patient or dispensed to said patient,
      ■ associating a unique identification number (UIDN) to said patient and associating said UIDN with said data packet, and
      ■ transmitting said UIDN along with said data packet to said remote computer
   ○ said remote computer ,
      ■ cross-referencing said drug products contained within said data packet with individual disease states
   ○ identifying disease state information that relates to said disease states

In another aspect of the invention, the present invention provides an automated system for providing health information to patients from one or more clinic computer systems or a component storing patient medical information, regardless of its operating system or software configuration, and without requirement by the system to encrypt data, comprising a clinic, physician, hospital or other computer system (generically referred to as a "clinic computer") configured to:
(a) store patient data, health information, health history and/or any archives of an individuals health record; and/or
(b) contain one or more event sequence identification data field(s) and/or diagnosis data field(s); and/or
(c) allow association of one or more unique identification numbers (UIDN) with a patient record; and/or
(d) allow the event sequence identification data field and/or diagnosis data field to be associated with one or more generated unique identification numbers (UIDN); and/or
(e) generate transactional data that associates selected patient record data fields, event sequence data field and/or diagnosis data field with the UIDN; and/or
(f) allow transfer of said transactional data over a local area network, wide area network or Internet or other coupling to one or more third party and/or remote computers or component containing libraries or databases of health information and/or third party payer health plan information; and/or
(g) allow the clinic computer to receive patient health information and associated UIDN that is transmitted from the third party and/or remote computer(s), or component; and/or
(h) store part or all of the health information and any other associated data transmitted from the third party and/or remote computer; or component; and/or
(i) re-associate the health information transmitted from the third party and/or remote computer system or component with the UIDN associated with the stored patient record; and/or
(j) print the health information received from the third party and/or remote computer system or component; and/or
(k) email, text message or otherwise make available either directly or through another computer the health information received from the third party and/or remote computer system or component; and/or
(l) print or stamp the patients name or assigned clinic number on the third party or remote or component health information so that clinicians may match it with any information provided by the clinic computer; and/or
(m) store a list of patients categorized by disease state and merge this list with a print or email newsletter related to the disease state.

In one embodiment of the invention, information can be transferred between computers using unencrypted means which overcomes barriers known in the art.

In yet another embodiment of the invention, information concerning the patient such as one or more than one of the following: patient's name, address, health number, government ID number, insurance ID number, clinic assigned patient number, birthday, admission date, discharge data and geographic data is removed from the data packet.

In yet another embodiment of the invention, the system is capable of running in real-time.

In yet another embodiment of the invention, the data packet will also comprise medical diagnosis data.

In yet another embodiment of the invention, the clinic and remote computers are connected via the internet, local area or wide area network.

In yet another embodiment of the invention, the clinic computer is attached to a printer configured to print out the information obtained from the remote computer.

In yet another embodiment of the invention, the remote computer also stores transactional frequency data for each UIDN and can use the stored data in future transactions.

In yet another embodiment, the disease state information will contain a unique identifier or password that would allow a patient to subsequently access information related to their health condition through a networked computer or the internet.

In yet another embodiment of the invention, the remote computer analyses the data packet and determines if the patient has complied with previously prescribed therapy, and if not, an additional message is added to the health information with the intent of improving compliance with the prescribed therapy.

In yet another embodiment of the invention, the provision of the health or medication history information to the patient is done through email, mail, text messaging, SMS or via the telephone.

In yet another embodiment of the invention, information could be sent directly to the patients location, if the patient is outside of the pharmacy or health institution, by the clinic computer or authorized agent while still protecting the patients identity from unauthorized third parties.

In yet another embodiment, the unique system described herein allows multiple variables to be considered and each resulting printout can be customized for each individual patient. Each printout consists of a series of sections (i.e., blocks) of text, with each section being composed of unique information, thereby re-defming the concept of a printout to be given to a patient. The printouts are the result of the system collecting a series of data and determining relevant information that is then used collectively to create a printout with multiple unique sections, each section containing information related to a particular aspect of health data stored in the computer system related to an individual patient. For example, other systems may recognize that a patient is taking a non-steroidal anti inflammatory drug (NSAID) but would not know if that medication was intended to treat arthritis or a sports injury (NSAID's treat both of these conditions). In this embodiment, the system described herein is able to determine the intended purpose of the medication (arthritis OR sports injury, not both) by analyzing previously administered drugs that had been administered and other information that is available and then provide a printout that contained multiple and related sections, each section containing unique information.

This ability to create a multivariable printout also allows for more complete and accurate information to be provided. For example, by recognizing a patient is taking a particular medication that caused vitamins or nutrients to be depleted by their body, information can be added to one block providing advice on how to prevent such nutrient depletion. In another way, if a medication is known to interact with other medications, another block can be added to the printout providing a warning for such an interaction. In this manner the printout is now tailored for each individual patient situation.

A system according to the invention may be one in which said health information is made available to said patient through email, mail, text messaging, SMS, MMS or telephone or through a print out from a printer connected to said clinic computer,

Said remote computer may generate and transmit to said clinic computer a unique distribution identification number (UDIDN) and transmit to a distribution computer said UDIDN and said health information, whereby said UDIDN is made available to said patient from said clinic computer; said patient may suitably access said health information from said distribution computer by referencing said UDIDN; the patient may access said health information through either email, text messaging, SMS, MMS or web browsing.

The system of the invention may be used in a health care institution; the data packet can also comprises medical diagnosis data.

Generally, clinic and remote computers are suitably connected via the internet. Also generally, clinic and remote computers may be connected over a local area or wide area network or over a non-encrypted data transfer link.

It is an option the the generation and transmission of said UIDN complies with government privacy regulations. Separately, in the process of generating said UIDN, information pertaining to at least one of the following items:
Name, Street Address, Health number, Government identification number, Insurance identification number, Clinic assigned patient number, Birth date, admission date and discharge date, geographic data,
   may be removed.

Further optionally, the clinic computer is configured to operate in real-time; said remote computer is configured to store said health information for later distribution to said patient.; and the remote computer determines if patients have not complied with their prescribed therapy based on said data packet and wherein said disease state information also comprises a message with the intent of improving compliance with prescribed therapy.

### BRIEF DESCRIPTION OF THE SEVERAL VIEWS OF DRAWINGS ILLUSTRATING EXEMPLARY EMBODIMENTS

The following figures illustrate preferred and alternative embodiments of the invention, wherein:
FIG. 1 is a high level schematic diagram for illustrating various exemplary embodiments of the present invention,
FIG. 2 illustrates an exemplary flow of data for certain exemplary embodiments of the invention.
FIG. 3 illustrates one example of the layout of a newsletter generated by certain exemplary embodiments of the invention.

### Detailed Description of Embodiments of the Invention

While the foregoing provides a detailed description of a preferred embodiment of the invention, it is to be understood that this description is illustrative only of the principles of the invention and not limitative. Furthermore, as many changes can be made to the invention without departing from the scope of the invention, it is intended that all material contained herein be interpreted as illustrative of the invention and not in a limiting sense.

In an embodiment of the invention, the system can be implemented in a wide variety of locations including hospitals, physician clinics, pharmacies, laboratories or any location where a patient record is stored or received. The system is designed to integrate into existing workflow patterns, provide the option for individual clinics to opt-in or opt-out, and provide the healthcare professional with tracking tools they can use to apply for reimbursement fees from governments or other health service payers. Such a system provides mechanisms to register patients for follow-up and ongoing support programs and allows for the integration of a healthcare professional education library so that physicians, pharmacists and nurses can educate themselves on how to best counsel patients on a condition.

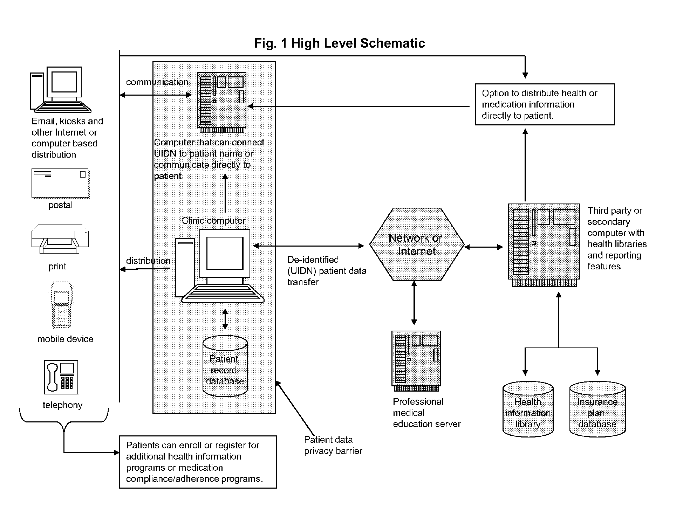

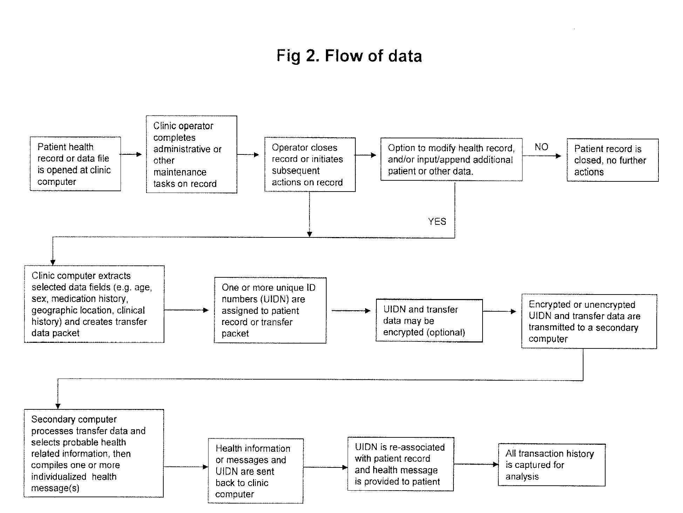

In Figure 1, a high level schematic showing the interoperation between clinic computer and a third part or secondary computer (i.e., remote computer) is depicted. In figure 2, a flow sheet of data is depicted that describes an embodiment of the invention. In these figures, a clinic computer is present that has access to medical information for patients located within a patient record database. The clinic computer need not be only a single computer but can be computer system or network of computers. In a preferred embodiment, the patient record database is located on the clinic computer itself so as to allow the clinic computer to perform an internal lookup of the patient's information. However, the database need not be located on the clinic computer itself. For example, the database can be located on external media- for example, an external hard drive where the clinic computer is configured to access the database. In addition, the database can also be physically located on another computer which is networked with the clinic computer to allow the retrieval by the clinic computer of relevant patient information. The clinic computer may also access patient information from more than one source (e.g. computer). The source of patient data may also itself transmit information on a particular patient to a memory component which may accumulate further data on a particular patient from additional sources. In another embodiment, the clinic computer can also be used for general patient record keeping or other computer related tasks not directly associated with the present invention. In another embodiment of the invention, the clinic computer can be used to improve patient management by health care professionals and provide a number of built-in professional level information resources. Such information can include, for example, diagnosis estimates, lab test reporting features and other patient charting options.

The patient record database will generally hold data, health information, health history and/or archives of health records particularized to an individual patient such as name, address, birth date, sex, health card number, etc. Depending on whether the system is being used in a pharmacy setting or institutional setting, the patient record database will also contain additional information for each patient. For example, in a pharmacy setting, the patient record database will generally contain information on previously prescribed drugs which can include what drugs were prescribed, how many drugs and when were the drugs dispensed. This type of information can be used to determine if a particular prescription is new or a refill and whether the patient is complying with a previously prescribed therapy. For example, if a patient is only seeking a refill several weeks after their previous supply would have run out, a determination can be made that the patient has been without drug therapy for those several weeks. In an institutional setting, the patient record database can also contain information about drugs that were prescribed (and potentially administered if in a hospital setting), but can also include other pertinent information about the patient such as diagnosis data related to conditions that the patient has been diagnosed with or procedures that the patient has undergone. Such information can be contained within event sequence identification data field(s) and/or diagnosis data field(s) as records that can be accessed by the clinic computer.

When a patient is interacting with a medical or pharmacy professional who uses the system of the current invention, the clinic computer will query the patient record database and obtain relevant drug related information about the patient. The clinic computer will then generate, assign and store a unique identifier number (UIDN) for the particular patient. The UIDN can be random or in one embodiment, the UIDN can be generated using an algorithm that combines information particularized to the individual patient (such as name, birth date, geographic location, etc.) in such a manner that the resulting UIDN does not identify any details about the patient and can not be reverse engineered to obtain the individual patient information. In such a manner, the UIDN generated for a given patient will always be the same, but nonetheless cannot be used by anyone who doesn't have access to the clinic computer to personally identify the patient. The generation of the UIDN allows the clinic computer to disassociate the patients name or other information that would identify the patient from any transmitted data to a third party or a remote or other computer or component.

The clinic computer then creates a data packet which contains information on the drugs that the patient is being prescribed and can include information such as the number of times it has been prescribed/dispensed, date of last dispensing, quantity of pills dispensed, number of days of medication supplied, potential refills remaining, the drug identification number, the drug therapeutic class, etc. Other patient information (if available) can also be included such as age and/or gender. It is also possible that the data packet may contain information about prescriptions that have been recently prescribed, such as those in the past year, though any time frame can be arbitrarily chosen; the information about recent prescriptions being similar to that being transferred about the current prescription. In a preferred embodiment for institutional settings, the data packet can also contain diagnosis data. The resulting data packet is then transmitted with the UIDN via a network connection (e.g., local area network or wide area network) or the internet to a remote database computer (also termed the secondary computer in Figure 2). The transmission of the data packet and UIDN can be done through encrypted or non-encrypted means since the information being transmitted (i.e., data packet and the UIDN) cannot themselves be used to personally identify an individual patient. In one embodiment, by being able to transmit information over unencrypted means, many pharmacies or health care institutions may provide information to a patient. Such institutions under normal circumstances would be unable to justify implementation of encryption technologies that would meet the U.S. Health Insurance Portability and Accountability Act or other patient health record privacy requirements.

The remote database computer's primary task is to take the information about the drug products from the data packets and use this information to determine likely disease states for the particular UIDN and locate and identify relevant disease state information about the disease state that is to be ultimately given to the patient. The database computer achieves this by accessing a drug-disease database that contains information on drug products and likely disease states. The drug-disease database can be physically located on the database computer itself or situated on external media or situated on another computer that is networked with the database computer. The database computer can use an algorithm which takes into account more than just the information pertaining to the drug products themselves. The diagnosis data and patient information (if available) can be used as can patient demographic data and information about past drug products that have been prescribed or dispensed. In a preferred embodiment, the database computer has storage means and can store transactional data relating to each UIDN and as a result, more information can be made available for subsequent information requests for a given UIDN.

In another embodiment of the invention, database computer can differentiate between chronic and acute conditions and can tailor the disease state information appropriately.

The disease state information relates to general information about a particular disease, independent of drug treatment and the individual topics can be used for a wide variety of drug products. For example, drugs used against ulcers such as proton pump inhibitors or H₂-receptor antagonists may lead to disease state information being produced on gastric or duodenal ulcers.

The disease state information is then assembled into health information to ultimately be distributed to the patient. The health information may also contain other messages or information not specifically related to diseases derived from the drugs being prescribed. For example, in one embodiment, the remote computer may be able to determine if a patient is complying with their prescribed therapy by analysis of the previous prescription and/or dispensing history for the patient either contained within the data packet or possibly stored independently in the remote computer for a given UIDN. If such a scenario arises, the health information can also include messages which remind the patient to take their medications with the intent of improving the patients compliance with the prescribed therapy. Another example is that if a male patient is determined to be of a certain age, a prostate examination may be recommended.

The remote database computer may also be used to perform other useful tasks. For example, the database computer can collect aggregate data on drugs being prescribed/dispensed and interface with insurance plan databases or health information libraries to allow the drug insurance companies or the general public to monitor what diseases are becoming prevalent. The remote database computer may also in another specific embodiment, store the health information for later access by the patient. In another embodiment, the health information may also be passed on to a third computer connected via network to the database computer for later distribution to the patient. In this embodiment, the third computer would contain an identifying label that could be distributed to the patient to allow the patient to access the information directly from the third computer.

In one embodiment, the remote database computer then transmits back to the clinic computer the health information along with the UIDN. The UIDN may not need to be transferred if the system is being used in real-time. The clinic computer takes the health information and determines which patient is to receive this information by cross-referencing the UIDN to the actual patients. The health information is either stored for later transmittal to be appended to other information scheduled to be distributed to the patient (or may be independently distributed to patients), or given directly to the patient. The information can be transmitted in any known means to distribute information in a human intelligible form and can include a computer terminal, email kiosks, mail, direct printout, text message, SMS, MMS or telephone. In one embodiment, the clinic computer is configured with a printer to be used to printout the health information.

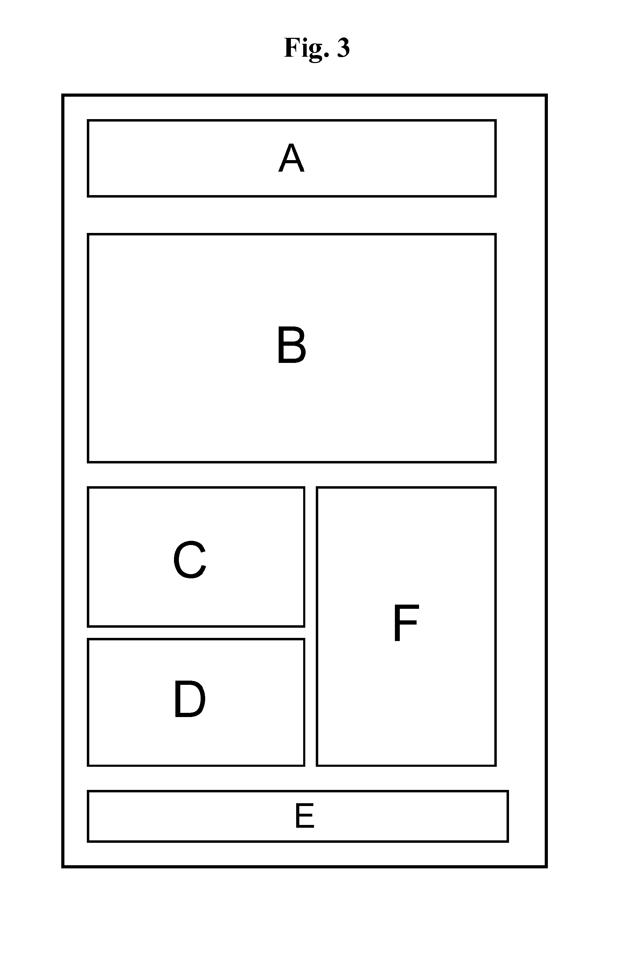

In another embodiment, the health information can then be used to generate a customized newsletter containing the various pieces of information. The information is then formatted into a newsletter which contains various sections. Figure 3 depicts an example of a newsletter that can be generated containing six discrete areas (i.e., blocks) containing the information. In other cases there may be only one or two blocks. The number of blocks to be used in the newsletter can be predetermined or automatically configured by the system depending on the amount of information that is determined to be relevant for a given patient. The individual blocks of information are assembled independently based on information contained in the data packet As one example, using the layout of figure 3, if the remote database computer was able to discern that the patient in question was being prescribed a cholesterol medication, Block A might have general information on managing cholesterol levels; Block B having general information related to cholesterol and high cholesterol and related symptoms. Block C can have custom information unique to an individual pharmacy that was suitable for that particular patient and not related to cholesterol such as flu shots and when the next flu short clinic is scheduled. Block D may contain health information unrelated to the medication taken based on the patients age and sex such as a warning to get an annual prostate exam (for males) or breast exam (for females). Block E can contain a specific drug interaction alert related to the medication. Block F can contain a warning and information that the cholesterol lowering drugs may cause nutrient depletion. This overall process allows for a highly customized and unique printout.

In another embodiment, the remote database computer need not pass the health information back to the clinic computer, but rather can pass a customized weblink or customized health information identifier back to the clinic computer. In this embodiment, the health information would either be stored on the database computer itself or passed to an additional server, along with the customized health information identifier or weblink. The patient would be given either a customized link or identifier or password that would allow access to the relevant health information via a direct network connection or over the internet. The patient may also enroll or register for additional health information at the link. Such embodiments allow healthcare providers to have ongoing communication with patients using a wide variety of communications media.

In another embodiment, an optional healthcare professional medical education library situated on a server can be interconnected with the system by known means. The clinic or remote database computer can pull relevant information from the professional medical education server for later distribution to a medical professional, such as a physician, nurse or pharmacist to better educate themselves of relevant information on conditions or other recent developments that may be relevant at that time (e.g., pandemic alerts, drug recalls, etc.)

The system preferably operates in real-time, in that the patient is able to receive the health information almost instantaneously when the system is being used. However, the system can also be used in a batch like manner where a series of different data packets associated with different UIDN are all transmitted from the clinic computer to the remote database computer at the same time, the database computer analyses each data packet and UIDN and compiles a series of health information, each set of information being associated with a particular UIDN which is then transmitted back to the clinic computer. The clinic computer can then cross-reference the UIDN thereby reassociating the UIDN with a specific patient record and store the relevant health information for the next time that the patient visits the pharmacy or health care institution.

In another embodiment, the patient is not given the health information at the time of interacting with the system, but rather is given a customized unique distribution identification number which can be a number or a code (UDIDN). The patient can themselves initiate the process of receiving the health information by sending the UDIDN to a particular website, email address or via text message to a specific phone number which will have access to the health information and the unique identifier code and be able to transmit the information directly to the patient. The UDIDN can also be transformed or can be directly included into a web browser link to be transmitted to the patient. In this manner, the patient can initiate the access to information from a remote location and allow the remote database computer to transmit the health information directly (or indirectly through a third computer) to the patient bypassing the clinic computer, the patient confidentiality still being maintained.

In another embodiment, an existing computer system used in either a pharmacy or institutional setting that contains stored patient health records can be adapted so as to allow the computer to deliver health information to patients or individuals as part of a healthcare provider workflow using e.g. a third party or remote computer or other computer or component configured with an extensive database of health information. Such a system may achieve valuable results e.g.: i) e.g., whereby encryption technology is not required to complete a transaction, and/or; ii) e.g. whereby interaction is feasible with computers using diverse operating systems, and/or; iii) e.g. whereby applications are not limited to any specific health provider or type of health clinic, and/or; iv) e.g. whereby ongoing communication and follow-up with patients is effective and efficient.

In another embodiment, pharmacy or clinic based systems as disclosed herein need not only capture patients who have filled a prescription, but may also capture patients who have not yet been prescribed a drug or have not filled the prescription (approximately 10% of prescriptions are never filled and 25% of prescriptions are not re-filled). For example, the pharmacy system can allow employers or health plan payers, who have a vested interest in improving health, to participate in selecting which patient information is distributed. Furthermore, exemplary embodiments provide the ability to capture patient contact information so that ongoing follow-up can be administered centrally.

In another embodiment, a patients or other third party may send a message or request to the system, containing the UIDN, for the purpose of retrieving the patients medication profile or other information contained within the system. For example, a patient who has visited an emergency room may send an SMS or email message containing their UIDN to the system, the system would then retrieve and send back the patient medication profile or other information such as the patients diagnosis or lab test results.

It can be appreciated that though the flow of data that has been depicted in the above embodiments is in a certain order, the order can nonetheless be modified. For example, in the embodiments described above, the data packet is created prior to the generation of the UIDN, it would be apparent to a person skilled in the art that these two events can be reversed.

It will be apparent that many modifications and variations may be effected without departing from the spirit and scope of the invention as defined within the attached claims.

## Claims

1. An automated system for providing health information to patients from one or more clinic computer systems or a component storing patient medical information, regardless of its operating system or software configuration, and without requirement by the system to encrypt data, comprising a clinic, physician, hospital or other computer system (generically referred to as a "clinic computer") configured to:
(a) store patient data, health information, health history and/or any archives of an individuals health record; and/or
(b) contain one or more event sequence identification data field(s) and/or diagnosis data field(s); and/or
(c) allow association of one or more unique identification numbers (UIDN) with a patient record; and/or
(d) allow the event sequence identification data field and/or diagnosis data field to be associated with one or more generated unique identification numbers (UIDN); and/or
(e) generate transactional data that associates selected patient record data fields, event sequence data field and/or diagnosis data field with the UIDN; and/or
(f) allow transfer of said transactional data over a local area network, wide area network or Internet or other coupling to one or more third party and/or remote computers or component containing libraries or databases of health information and/or third party payer health plan information; and/or
(g) allow the clinic computer to receive patient health information and associated UIDN that is transmitted from the third party and/or remote computer(s), or component; and/or
(h) store part or all of the health information and any other associated data transmitted from the third party and/or remote computer; or component; and/or
(i) re-associate the health information transmitted from the third party and/or remote computer system or component with the UIDN associated with the stored patient record; and/or
(j) print the health information received from the third party and/or remote computer system or component; and/or
(k) email, text message or otherwise make available either directly or through another computer the health information received from the third party and/or remote computer system or component; and/or
(l) print or stamp the patients name or assigned clinic number on the third party or remote or component health information so that clinicians may match it with any information provided by the clinic computer; and/or
(m) store a list of patients categorized by disease state and merge this list with a print or email newsletter related to the disease state.

2. A system according to claim 1, wherein said clinic computer system is configured to deidentify a patient record in compliance to government privacy regulations and create an unencrypted unique identification number (UIDN).

3. A system according to claim 1 or 2, wherein said clinic computer system is configured to create de-identified transactional data from a patient health record by associating the UIDN with each stored patient record and remove from said transaction data at least one of the following items: name, street address, health number, any government identification number(s), any insurance identification number(s), clinic assigned patient number(s), birth date, admission date, discharge dates, geographic data.

4. A system according to any of claims 1 to 3, wherein said clinic computer system is configured to provide the option for the operator of the clinic computer to opt-in or opt-out of sending the transactional data through default mechanisms or through real-time prompts displayed on the clinic computer screen or other prompting mechanism and further configured to transmit said unique identifier to a central and/or remote computer system and/or third party computer system (the "third party computer system") and that system can store the unique identifier.

5. A system according to any of claims 1 to 4, further comprising a printer connected to the clinic computer which is configured to instruct said printer to print said patient information.

6. A system according to any of claims 1 to 5 wherein the clinic computer is configured to transmit the UIDN and transfer data, and further comprising a secondary, third party and/or remote, and/or other computer system or component configured to receive the transmitted data from the clinic computer.

7. A system according to claim 6, wherein said third party and/or remote, and/or other computer system is configured to select one or more health messages based on the transmitted data.

8. A system according to claim 6, wherein the third party and/or remote, and/or other computer system or component has the option to store transactional frequency data for each UIDN or purge all or portions of said data for each transaction.

9. A system according to claim 6, wherein said third party and/or remote, and/or other computer system or component is configured to transmit to the clinic computer one or more said informational message(s) in association with the UIDN or encrypted patient data.

10. A system according to any of claims 1 to 9 wherein said clinic computer can print one or more unique identifiers or passwords on the patient printout and the patient may subsequently access transactional data or additional information related to their health condition through a networked computer or the internet.

11. A system according to claim 6 wherein the third party and/or remote, and/or other computer system or component can further determine if patients have been compliant with their prescribed therapy based on transmitted data and wherein said system tailors the transmitted message to the clinic computer with the intent of improving compliance with prescribed therapy.

12. A system according to claim 6 wherein the third party and/or remote, and/or other computer system or component can send compliance messages or reminders to patients via email, mail, wireless, text messaging, SMS or telephony.

13. A system according to claim 6 wherein third party and/or remote, and/or other computer system or component can provide reports on disease trends at individual clinics and suggest professional level medical education to clinic staff that is tailored to address the health needs of their specific patient population.

14. A system according to claim 6 wherein the third party and/or remote, and/or other computer system or component can generate analysis reports that can be used by clinic staff to claim fees for counseling services provided.

15. A system according to any previous claim, said system for use by pharmacies, clinics or health care institutions, said system for providing health information to a patient, said system comprising:
- a clinic computer having access to medical information for said patient, said medical information comprising at least a medication profile for said patient,
- a remote computer having access to a drug-disease database
said clinic and remote computers being networkably connected so as to allow data transfer between said computers,
- said health information comprising disease state information, said disease state information being determined by
○ said clinic computer,
■ analyzing said medication information for said patient
■ creating a data packet comprising drug products being used by said patient or dispensed to said patient,
■ associating a unique identification number (UIDN) to said patient and associating said UIDN with said data packet, and
■ transmitting said UIDN along with said data packet to said remote computer
○ said remote computer ,
■ cross-referencing said drug products contained within said data packet with individual disease states
■ identifying disease state information that relates to said disease states

16. A system according to claim 15 wherein said health information is transmitted from said remote computer back to said clinic computer, said clinic computer associating said health information with said patient and said health information being made available to said patient.

17. The system of any of claims 15 or 16 wherein the health information is formatted onto a page, said format comprising one or more blocks of information, each of said blocks of information containing a discrete piece of health related information customized for said patient.
